(19) Europäisches Patentamt

European Patent Office

Office européen des brevets

(11) EP 0 757 574 B1

(12) **EUROPÄISCHE PATENTSCHRIFT**

(45) Veröffentlichungstag und Bekanntmachung des Hinweises auf die Patenterteilung:
**29.05.2002 Patentblatt 2002/22**

(21) Anmeldenummer: **96903922.1**

(22) Anmeldetag: **26.02.1996**

(51) Int Cl.⁷: **A61N 1/05**

(86) Internationale Anmeldenummer:
**PCT/DE96/00350**

(87) Internationale Veröffentlichungsnummer:
**WO 96/25973 (29.08.1996 Gazette 1996/39)**

(54) **STIMULATIONSSYSTEM**

STIMULATION SYSTEM

SYSTEME DE STIMULATION

(84) Benannte Vertragsstaaten:
**DE FR GB NL**

(30) Priorität: **24.02.1995 DE 19507929**

(43) Veröffentlichungstag der Anmeldung:
**12.02.1997 Patentblatt 1997/07**

(73) Patentinhaber: **BIOTRONIK Mess- und Therapiegeräte GmbH & Co Ingenieurbüro Berlin 12359 Berlin (DE)**

(72) Erfinder:
• **BOIZ, Armin**
**D-91058 Erlangen (DE)**

• **SCHALDACH, Max**
**D-91054 Erlangen (DE)**
• **WETZIG, Thomas**
**D-91088 Bubenreuth (DE)**

(74) Vertreter: **Christiansen, Henning et al Eisenführ, Speiser & Partner Patentanwälte Pacelliallee 43/45 14195 Berlin (DE)**

(56) Entgegenhaltungen:
| | |
|---|---|
| EP-A- 0 500 215 | WO-A-92/21278 |
| WO-A-94/00050 | WO-A-94/06507 |
| DE-A- 4 207 368 | DE-A- 4 231 603 |
| US-A- 4 955 382 | |

**Beschreibung**

Die Erfindung betrifft ein Stimulationssystem gemäß dem Oberbegriff des Anspruchs 1.

Vielfältige elektrophysiologische Untersuchungen des monophasischen Aktionspotentials (MAP) zeigen, dass dieses den zeitlichen Verlauf des Transmembranpotentials mit großer Genauigkeit widerspiegelt. Im Gegensatz zur Messung des Transmembranpotentials, welche nur an isolierten Gewebeproben möglich ist, lässt sich das MAP mittels am Myokard anliegender Elektroden messen. Im Hinblick auf eine breite klinische Anwendung eröffnet sich damit der Zugang zu den in diesem Signal enthaltenen umfangreichen Informationen über den Zustand der Myokardzellen. Die Änderung der Morphologie des MAP unter dem Einfluss antiarrhythmischer Medikamente oder in Abhängigkeit von der Herzfrequenz eröffnet vielfältige Möglichkeiten für die breite klinische Diagnostik und Therapie unter anderem im Rahmen einer Schrittmachertherapie des Herzens. Gerade die Schrittmachertherapie als Langzeitbehandlung über Zeiträume von teilweise mehr als 10 Jahren stellt besonders hohe Anforderungen an die Elektrode als Sensor des MAP.

In HARDMAN, YOUNG, WALKER, BIGGS, SEED, NOBLE: A MULTI- ELEC-TRODE CATHETER FOR SIMULTANEOUS PACING AND REGISTRATION OF ENDOCARDIAL MONOPHASIC ACTION POTENTIAL, Europ. Jour. C.P.E., 1991, 2, Vol.I:75-82 ist eine endokardiale Elektrodenanordnung beschrieben, die sowohl die Messung des monophasischen Aktionspotentials als auch die Stimulation des Herzens erlaubt und aus vier Elektroden besteht, von denen zwei zur Stimulation des Herzens und zwei zur Messung des MAPs dienen.

Die Oberflächen der zur Messung des MAPs bestimmten Elektroden bestehen hierbei aus Ag/AgCl, um eine große Phasengrenzkapazität und eine geringe Phasengrenzimpedanz zu erreichen. Dadurch kann wegen der vernachlässigbaren Polarisierbarkeit eine ungestörte DC-gekoppelte Messung des MAP erfolgen. Die Stimulationselektroden weisen dagegen eine polierte Oberfläche auf.

Ein schwerwiegender Nachteil der beschriebenen Elektrodenanordnung ist darin zu sehen, dass die Silberchloridschicht der Ag/AgCl-Elektroden bei Kontakt mit biologischem Gewebe nicht langzeitstabil ist und das gelöste Silberchlorid aufgrund seiner Toxizität zu entzündlichen Reaktionen des umliegenden Gewebes führt.

Die beschriebene Elektrodenanordnung eignet sich also nicht zu einer dauerhaften intrakorporalen Messung des monophasischen Aktionspotentials.

Darüber hinaus ist bei der beschriebenen Elektrodenanordnung eine Trennung der Elektroden für die Stimulation des Herzens von den Elektroden für die Messung des MAPs erforderlich, da andernfalls das auf einen Stimulationsimpuls des Herzschrittmachers folgende Nachpotential die Messung des MAPs verfälschen würde. So wirkt die Grenzschicht zwischen Elektrode und dem umgebenden Gewebe als Kondensator mit einer bestimmten Kapazität. Bei einem Stimulationsimpuls fließt nun über diese Grenzschicht ein elektrischer Strom, der die als Kondensator wirkende Grenzschicht auf lädt, sodass über der Grenzschicht nach dem Abklingen des Stimulationsimpulses eine elektrische Spannung anliegt. Die Grenzschicht wird zwar nach dem Ende des Stimulationsimpulses wieder entladen, allerdings verfälscht dieses sogenannte Nachpotential die Messung des MAPs.

Ein Nachteil der beschriebenen Elektrodenanordnung ist deshalb darin zu sehen, dass vier Elektroden und entsprechend vier Zuleitungen erforderlich sind.

Die WO94/06507 offenbart die technische Lehre, bei einer Elektrodenanordnung die Oberfläche fraktal auszubilden oder mit einer elektroaktiven Beschichtung zu versehen, um die Phasengrenzimpedanz zu verringern und damit die Messung des MAPs zu ermöglichen. Eine solche Lehre ist auch in DE 42 07 368 beschrieben.

Der Begriff Elektrodenanordnung ist hierbei allgemein zu verstehen und umfasst sowohl Elektrodenanordnungen zur dauerhaften Stimulation im Rahmen einer Schrittmachertherapie als auch temporär einsetzbare Elektrodenanordnungen. Auch ist die Lehre nicht auf solche Elektrodenanordnungen beschränkt, die zur Herzstimulation dienen, sondern auch bei der Bekämpfung von Tachykardiezuständen anwendbar.

Die Elektrodenanordnung weist zwei Elektroden auf, von denen die eine - auch als differente Elektrode bezeichnet - das Herz elektrisch unmittelbar kontaktiert, während die andere - auch als indifferente Elektrode bezeichnet - das umliegende Gewebe elektrisch kontaktiert. Wichtig ist hierbei, dass die beiden Elektroden - abgesehen von der Verbindung durch das dazwischen gelegene Gewebe - elektrisch gegeneinander isoliert sind und somit eine bipolare Elektrodenanordnung bilden.

In einer Variante weist mindestens eine Elektrode eine fraktale Oberflächenstruktur auf, welche die wirksame Oberfläche der Elektrode vergrößert und damit die Phasengrenzimpedanz verringert. Die fraktale Oberflächenstruktur kann beispielsweise mit dem bekannten Magnetron-Kathodenzerstäubungsverfahren hergestellt werden. Hierbei wird auf einen vorzugsweise aus Titan bestehenden Träger eine Beschichtung aus Iridium aufgebracht, die eine fraktale Oberflächenstruktur aufweist. Der Werkstoff Titan eignet sich als Trägermaterial hervorragend wegen der guten Bioverträglichkeit, d. h. Titan wird vom Körper nicht abgestoßen und bei Kontakt mit menschlichem Gewebe auch nicht zersetzt.

Bei der Beschichtung des Trägers werden beispielsweise auf eine halbkugelförmige Elektrodenoberfläche zahlreiche kleine Halbkugeln aus dem Beschichtungsmaterial aufgebracht. Halbkugelförmige Elektrodenoberflächen sind z. B. in WO92/21278 offenbart. Hierdurch lässt sich die aktive Oberfläche der Elektrode in einem ersten Schritt verdoppeln. Auf die Oberfläche dieser Halbkugeln werden dann jeweils zahlreiche noch kleinere Halbkugeln aufgebracht. In solchen Schritten mit jeweils kleineren Halbkugeln als im vorangegangenen Schritt lässt sich die aktive Oberfläche der Elektrode auf das $2^n$-fache vergrößern.

Bei der Herstellung der fraktalen Oberflächenstruktur ist es nicht erforderlich, dass jeweils Halbkugeln aufgebracht werden. Vielmehr lassen sich die fraktalen Elemente mit nahezu allen geometrischen Formen bilden. Entscheidend ist, dass die fraktalen Elemente einer Schicht jeweils auf die Oberfläche der fraktalen Elemente der unmittelbar darunterliegenden Schicht aufgebracht werden, wobei die Größe der fraktalen Elemente nach außen hin abnimmt.

Durch die fraktale Oberflächenstruktur der Elektrode ist die aktive Oberfläche der Elektrode gegenüber einer gleich großen herkömmlichen Elektrode auf mehr als das tausendfache vergrößert, was vorteilhaft zu einer entsprechend verringerten Phasengrenzimpedanz führt.

An der Oberfläche der Elektrode bildet sich wegen des Dipolcharakters des Wassermoleküls eine Monolage aus Wassermolekülen. So werden in Abhängigkeit von der Polarität der Elektrode entweder die Wasserstoffatome oder die Sauerstoffatome von der Elektrode angezogen bzw. abgestoßen.

Die Grenzschicht zwischen einer Elektrode und dem umliegenden, vorwiegend aus Wasser bestehenden Gewebe lässt sich somit als Plattenkondensator betrachten mit der Elektrodenoberfläche als der einen Platte, dem Elektrolyten als der anderen Platte und der dazwischenliegenden Monolage aus Wassermolekülen als Dielektrikum.

Der Stromtransport zwischen der Elektrode und dem Gewebe erfolgt bei den fraktalen Elektroden vorwiegend kapazitiv. Beim Anlegen einer Spannung zwischen der Elektrode und dem Elektrolyten kommt es zu einem dielektrischen Verschiebungsstrom. Der Übergangswiderstand zwischen der Elektrode und dem umliegenden Gewebe hängt also von der Phasengrenzkapazität ab, die sich bekanntermaßen nach folgender Formel berechnet:

$$C_H = \varepsilon \cdot \varepsilon_0 \cdot \frac{A}{d}$$

Da die fraktalen Elektroden eine sehr große Oberfläche A aufweisen, ist auch deren Phasengrenzkapazität groß und damit die Phasengrenzimpedanz sehr klein. Deshalb sind bei MAP-Messungen mit den fraktalen Elektroden unter Verwendung von Messschaltungen mit Eingangswiderständen von mehr als einigen 10 kΩ Verfälschungen des Messsignals durch das Übertragungsverhalten der Phasengrenze vernachlässigbar. Mit den fraktalen Elektroden lässt sich deshalb vorteilhaft eine relativ große Empfindlichkeit bei der Messung des MAPs auch bei niedrigen Frequenzen erreichen. Darüber hinaus zeichnen sich fraktale Elektroden auf Grund ihrer niedrigen Phasengrenzimpedanz durch ein ausgezeichnetes Signal-Rausch-Verhältnis aus, was insbesondere bei einer Signalformanalyse des MAPs vorteilhaft ist.

Darüber hinaus folgt aus der Vergrößerung der aktiven Elektrodenoberfläche eine Verringerung des störenden Einflusses des Nachpotentials im Anschluss an einen Stimulationsimpuls. Bei der Stimulierung des Herzens durch einen Herzschrittmacher fließt während der Dauer des Stimulationsimpulses ein elektrischer Strom über die Phasengrenze zwischen der Elektrode und dem umliegenden Gewebe. Dabei wird die Phasengrenzkapazität aufgeladen. Nach dem Ende des Stimulationsimpulses wird die Phasengrenzkapazität zwar wieder entladen, allerdings verfälscht das Nachpotential während der Entladedauer die Messung des MAPs.

Die Spannung, auf die die Phasengrenzkapazität aufgeladen wird, berechnet sich dabei nach folgender, ebenfalls allgemein bekannter Formel:

$$U = \frac{1}{C_H} \int_0^T I(t)\, dt,$$

wobei I der kapazitive Strom über die Phasengrenze und $C_H$ die Phasengrenzkapazität ist. Die fraktalen Elektroden weisen nun eine extrem große Oberfläche und damit auch eine extrem große Phasengrenzkapazität auf. Aus der obenstehenden Gleichung ist ersichtlich, dass die Spannung, auf die die Phasengrenze aufgeladen wird, bei einer großen Phasengrenzkapazität sehr klein ist. Fraktale Elektroden mit einer großen Oberfläche weisen deshalb nur ein sehr geringes störendes Nachpotential auf. Aus diesem Grund kann die Stimulation des Herzens und die Messung des MAPs bei Verwendung fraktaler Elektroden vorteilhaft mit demselben Elektrodenpaar erfolgen.

Die fraktalen Elektroden weisen also einen anderen Stromtransportmechanismus auf als die vorbekannten Ag/AgCl-Elektroden.

Dies ermöglicht eine dauerhafte Verwendung der Elektrodenanordnung insbesondere in Verbindung mit einem ANS-gesteuerten Herzschrittmacher. So lassen sich aus der Morphologie des MAPs Informationen über die Aktivität des autonomen Nervensystems (ANS) gewinnen, sodass das MAP zur Ratensteuerung des Herzschrittmachers herangezogen werden kann.

Ein weiterer Vorteil der fraktalen Elektroden ist darin zu sehen, dass die Empfindlichkeit gegenüber Änderungen des Elektrodenandrucks wesentlich geringer ausgeprägt ist als bei den eingangs beschriebenen vorbekannten Ag/AgCl-Elektroden, was die Verwendung großflächiger Elektroden ermöglicht.

In einer anderen Variante ist dagegen vorgesehen, mindestens eine der beiden Elektroden zur Verringerung der Phasengrenzimpedanz insbesondere bei niedrigen Frequenzen mit einer elektroaktiven Beschichtung zu versehen, die beispielsweise aus einem iridiumoxid-haltigen Material bestehen kann.

In einer Variante sind die beiden Elektroden zu jeweils einer Symmetrieachse symmetrisch ausgebildet und so angeordnet, dass die Symmetrieachsen der beiden Elektroden im wesentlichen fluchten. Hierdurch wird der störende Einfluss von spontan erregten IEKG-Signalen bzw. evozierten Myokardpotentialen auf die Messung des MAP weiter minimiert.

Bei der Kontaktierung von menschlichem Gewebe hängt der Übergangswiderstand vom Anpressdruck der Elektrode ab. Diese Abhängigkeit ist bei fraktalen Elektroden zwar - wie vor stehend erwähnt - geringer ausgeprägt als bei Ag/AgCl-Elektroden, wirkt sich jedoch nach wie vor aus.

Bei einem großen Anpressdruck wird eine gute Formanpassung von Elektrode und Gewebe und damit ein geringer Übergangswiderstand erreicht. Dies spricht zunächst für eine möglichst spitze Form der Elektrode, da sich hierbei die Anpresskraft auf eine kleine Fläche verteilt und somit ein hoher Anpressdruck erreicht wird.

Andererseits ist der Übergangswiderstand von der wirksamen Kontaktfläche zwischen Gewebe und Elektrode abhängig. So weisen Elektroden mit einer großen Kontaktfläche eine relativ geringen Übergangswiderstand auf. Dies spricht für eine flächige Form der Elektrode.

In einer Variante ist deshalb eine Elektrode im wesentlichen halbkugelförmig oder halbkugelschalenförmig ausgeführt, es sind jedoch auch andere Kugelschnitte oder Kugelschalenschnitte als Form der Elektrode denkbar. Eine derartige Form stellt einen guten Kompromiss dar zwischen der Forderung eines hohen Anpressdrucks und damit einer möglichst spitzen Form einerseits und der Forderung nach einer großen Kontaktfläche und damit einer möglichst flächigen Form andererseits. Die halbkugelförmige Ausführung einer Elektrode ist z. B. aus WO92/21278 bekannt.

Bei der Elektrodenanordnung dient eine Elektrode bei der Messung des monophasischen Aktionspotentials zur unmittelbaren Kontaktierung des Herzmuskels, während die andere Elektrode als Gegenelektrode das umliegende Gewebe kontaktiert. Da das Potential der den Herzmuskel kontaktierenden Elektrode durch dessen Potential bestimmt ist, wird diese Elektrode auch als differente Elektrode bezeichnet. Das Potential der anderen Elektrode ist dagegen auch von dem Stromfluss zwischen dieser Elektrode und der differenten Elektrode abhängig. Das Potential der anderen Elektrode "schwimmt" also. Diese Elektrode wird deshalb auch als indifferente Elektrode bezeichnet.

In einer Variante sind die beiden fraktalen Elektroden in einem Abstand von im wesentlichen fünf Millimetern zueinander angeordnet. Bei einer derartigen Anordnung der Elektroden werden vorteilhaft sowohl bei Spontanerregung als auch bei Stimulation MAPs gleicher Morphologie gemessen, sodass die Messung des MAPs durch die Abgabe eines Stimulationsimpulses nicht oder nur unwesentlich verfälscht wird. Ein Abstand von 5 mm zwischen den Elektroden ist z.B. in US 4,955,382 oder WO92/21278 beschrieben.

In einer Variante ist die Elektrodenanordnung aus dem gleichen Grund so angeordnet, dass die differente Elektrode das Messobjekt, beispielsweise den Herzmuskel, kontaktiert, während die indifferente Elektrode bezüglich der Myokardoberfläche senkrecht über der differenten Elektrode angeordnet ist. Hierdurch wird vorteilhaft der störende Einfluss spontan erregter IEKG-Signale bzw. evozierter Myokardpotentiale minimiert.

Herzschrittmachersysteme mit fraktalen oder konventionellen Elektroden zur Abgabe von Steuerimpulsen und zur Aufnahme von das Stimulationsverhalten beeinflussenden Steuersignalen sowie einer Eingangsstufe, die eingangsseitig zur Aufnahme des evozierten Potentials mit der Elektrodenanordnung verbunden ist und ausgangsseitig zur Steuerung des Impulsgenerators des Herzschrittmachersystems mit dem Steuereingang des Impulsgenerators verbunden ist, sind z. B. in DE 42 31 603 offenbart.

Gegenüber diesem Stand der Technik besteht die Aufgabe der vorliegenden Erfindung darin, ein Stimulationssystem bereitzustellen, das ohne zusätzliche Elektroden eine verbesserte Steuerung des Impulsgenerators ermöglicht.

Diese Aufgabe wird durch ein Stimulationssystem nach Anspruch 1 gelöst.

Weitere vorteilhafte Ausgestaltungen der vorliegenden Erfindung sind in den Unteransprüchen angegeben.

Das MAP enthält - wie bereits eingangs erwähnt - vielfältige Informationen, wie beispielsweise Sympathikus- und Parasympathikustonus (Informationen des autonomen Nervensystems), die im Rahmen einer Schrittmachertherapie zur Optimierung des Schrittmacherverhaltens ausgewertet werden können und eine ANS-Steuerung der Schrittmacher-Rate ermöglichen. Darüber hinaus enthält das MAP Informationen über sich anbahnende Arrhythmien, was eine rechtzeitige Bekämpfung von Tachykardiezuständen ermöglicht.

Zur Stimulation des Herzens ist ein Impulsgenerator vorgesehen, der zur Abgabe von Stimulationsimpulsen mit mindestens einer der Elektroden der Elektrodenanordnung verbunden ist. Die Stimulation des Herzens kann also wahlweise unipolar oder bipolar erfolgen. Zur Beeinflussung des Stimulationsverhaltens des Impulsgenerators weist dieser einen Steuereingang auf. Auf diese Weise ist es beispielsweise möglich, das Escape-Intervall des Herzschrittmacher bei einer Belastung des Herzschrittmacherträgers entsprechend anzupassen. Die Erfindung ist jedoch nicht auf die Anpassung des Escape-Intervalls beschränkt. Vielmehr ist es möglich, verschiedene Betriebsparameter des Herzschrittmachers an die Belastung anzupassen.

Da das über die Elektroden aufgenommene Messsignal neben dem monophasischen Aktionspotential auch hochfrequente Störungen sowie die von spontanen Herzaktionen herrührenden elektrischen Signale enthält, sind gemäß der Erfindung Mittel vorgesehen, um das MAP aus dem über die beiden Elektroden aufgenommenen Messsignal zu isolieren. In einer bevorzugten Ausführungsform weisen diese Mittel ein Bandpassfilter auf, dessen obere Grenzfrequenz vorzugsweise kleiner ist als 5000 Hz. Die untere Grenzfrequenz sollte dagegen unterhalb von 0,5 Hz liegen, um auch niederfrequente Signalanteile auswerten zu können. Statt des Bandpassfilters kann auch ein Tiefpassfilter verwendet werden. Die untere Grenze des Bandpass- oder des Tiefpassfilters liegt vorzugsweise zwischen 0,1 und 0,5 Hz, insbesondere zwischen 0,25 und 0,5 Hz.

Darüber hinaus ist es jedoch auch möglich, Korrelationsfilter oder andere Signalverarbeitungstechniken zu verwenden, um das MAP aus dem Messsignal zu isolieren.

Weiterhin sind gemäß der Erfindung Mittel vorgesehen, um aus dem MAP den physiologisch angepassten Wert der Steuergröße zu ermitteln. Vorzugsweise wirkt sich das MAP auf die Stimulationsrate aus, um unter Berücksichtigung der jeweiligen physischen oder psychischen Belastung des Herzschrittmacherträgers eine ausreichende Herzleistung sicherzustellen. Es hat sich hierbei gezeigt, dass die physiologisch sinnvolle Herzrate aus der Zeitdauer des MAPs ermittelt werden kann. In einer Variante der Erfindung sind deshalb Mittel vorgesehen, um die Zeitdauer des MAPs bzw. die Breite des MAP-lmpulses zu bestimmen. Der auf diese Weise ermittelte Wert wird dann einer Zuordnungseinheit zugeführt, die jedem Wert der MAP-Dauer entweder direkt einen Wert der Stimulationsrate oder einen Änderungswert der Stimulationsrate zuordnet, wobei der Änderungswert angibt, um welchen Wert der aktuelle Wert der Stimulationsrate verändert werden soll. In Abhängigkeit davon wird dann das Steuersignal erzeugt, das den Impulsgenerator ansteuert.

Im Betrieb des Herzschrittmachers kann das über die Elektrodenanordnung abgenommene Messsignal des MAP Schwankungen unterliegen, die nicht aktivitätsabhängig sind, sondern beispielsweise aus einer geringfügig veränderten Position der Elektrodenanordnung im Körper des Herzschrittmacherträgers oder Alterungserscheinungen des Herzschrittmacherträgers resultieren. Da eine Auswirkung derartiger Schwankungen auf die Stimulationsrate des Herzschrittmachers unerwünscht ist und die Aktivität des autonomen Nervensystems des Herzschrittmacherträgers in besonderem Maße die Morphologie des MAP beeinflusst, wird in einer bevorzugten Ausführungsform dieser Variante nicht die Größe des MAPs, sondern dessen Morphologie zur Ratensteuerung verwendet.

Eine Möglichkeit zur Erfassung der Morphologie des MAPs besteht darin, das MAP einer Fourier-Analyse zu unterziehen und auf diese Weise dessen Frequenzspektrum zu ermitteln. Die angemessene Herzrate ergibt sich dann aus dem Wert des Frequenzgangs für bestimmte Frequenzen, bei denen der Frequenzgang eine besonders signifikante Abhängigkeit von der Aktivität des autonomen Nervensystems des Herzschrittmacherträgers zeigt.

In einer anderen Ausführungsform ist dagegen vorgesehen, das Messsignal des MAPs abzutasten und die Abtastwerte mit vorgegebenen Werten zu vergleichen. Da das MAP entsprechend der jeweiligen Herzrate periodisch ist, wird hierbei jeweils ein bestimmtes Zeitfenster innerhalb eines Herzzyklus' betrachtet, um die Auswirkungen der periodischen Schwankung des MAPs auf die Messung zu unterdrücken. Die angemessene Herzrate ergibt sich dann aus den Amplituden der einzelnen Abtastwerte, die die Morphologie des MAPs widerspiegeln.

Von großer therapeutischer Bedeutung ist die Bestimmung der MAP-Dauer bei verschiedenen Repolarisationsstufen.

Das MAP enthält - wie bereits vorstehend erwähnt - Informationen über sich anbahnende Tachykardiezustände. In einer vorteilhaften Variante der Erfindung ist deshalb vorgesehen, das MAP einer Signalverarbeitungseinheit zuzuführen, die das MAP analysiert und beurteilt, ob ein Tachykardiezustand bevorsteht. In einem derartigen Fall gibt die Signalverarbeitungseinheit ein Steuersignal an den Impulsgenerator oder einen Defibrillator, der daraufhin in eine besondere Betriebsart wechselt, die die Prävention oder Bekämpfung der Tachykardie in an sich bekannter Weise ermöglicht.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

| | |
|---|---|
| Figur 1a bis 1d | verschiedene Ausführungsformen von Elektrodenanordnungen mit zwei bis sieben Elektroden, |
| Figur 2a bis 2e | mehrere bipolare Elektrodenanordnungen, |
| Figur 3a bis 3c | eine Prinzipdarstellung einer fraktalen Oberflächenstruktur in verschiedenen Stadien des Herstellungsprozesses, |
| Figur 4 | ein Stimulationssystem als Blockschaltbild, sowie |
| Figur 5a bis 5f | jeweils ein Diagramm eines monophasischen Aktionspotentials. |

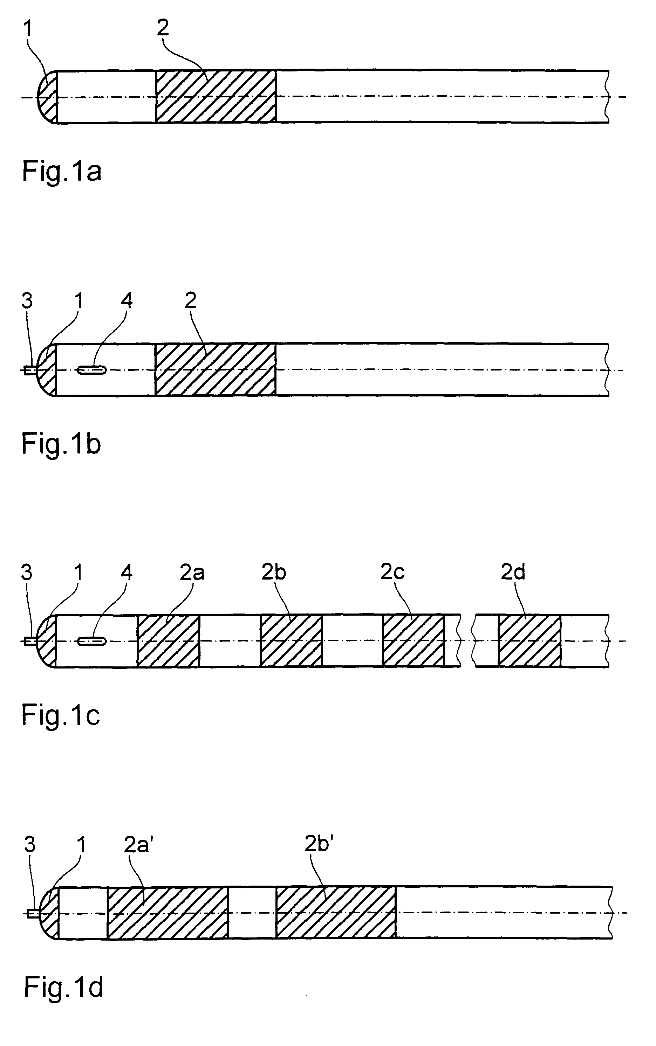

Figur 1a zeigt als Ausführungsbeispiel der in der Erfindung verwendeten Elektrodenanordnung eine implantierbare bipolare Elektrodenanordnung, die im wesentlichen aus einer halbkugelschalenförmigen ersten Elektrode 1 und einer ringförmigen zweiten Elektrode 2 besteht, die so angeordnet sind, dass deren Symmetrieachsen im wesentlichen fluchten. Der Abstand zwischen den beiden Elektroden 1, 2 beträgt ca. 5 mm. Die Länge jeder Elektrode kann z. B. 6 mm betragen. Durch diese Anordnung wird vorteilhaft erreicht, dass bei der Messung des MAPs sowohl bei Spontanerregung als auch bei Stimulation des Herzens das MAP die gleiche Morphologie aufweist. Dies ist wichtig bei der Auswertung des zeitlichen Verlaufs des MAPs bei der klinischen Diagnostik beispielsweise von Arrythmien des Herzens.

Die beiden Elektroden 1, 2 bestehen jeweils aus einem Träger aus Titan und einer Beschichtung aus Iridium. Die Verwendung von Titan als Material für den Träger ist vorteilhaft, da Titan bioverträglich ist, d. h. Titan wird vom Körper nicht abgestoßen und geht auch keine chemischen Reaktionen mit dem Körpergewebe ein. Die Beschichtung mit Iridium hat die Funktion, die aktive Oberfläche der Elektroden 1, 2 zu vergrößern. So werden beispielsweise auf die halbkugelschalenförmige Elektrode 1 zahlreiche aus Iridium bestehende Halbkugeln aufgebracht, wobei der Radius dieser Halbkugeln wesentlich geringer ist als der Radius der Elektrode 1 selbst. Auf diese Halbkugeln werden dann wiederum aus Iridium bestehende, noch kleinere Halbkugeln aufgebracht, wodurch die aktive Oberfläche der Elektrode 1 abermals vergrößert wird. Durch die n-malige Wiederholung dieses Schritts in einem zusammenhängenden Aufwachsprozess mit immer kleineren Halbkugeln lässt sich so die aktive Oberfläche der Elektrode 1 auf das $2^n$-fache steigern.

In der gleichen Weise wird die aktive Oberfläche der ringförmigen zweiten Elektrode 2 vergrößert.

Die halbkugelschalenförmige differente Elektrode 1 dient bei der Messung des MAPs zur unmittelbaren Kontaktierung des Herzmuskels, während die ringförmig ausgebildete indifferente Elektrode 2 das die differente Elektrode 1 umgebende Gewebe kontaktiert.

Figur 1b zeigt als weiteres Ausführungsbeispiel der Elektrodenanordnung eine 4-polige Elektrodenanordnung, die sich insbesondere zur Messung des MAPs eignet.

Die Elektrodenanordnung weist - wie die bereits in Figur 1a dargestellte Elektrodenanordnung - eine halbkugelschalenförmige erste Elektrode 1 zur Kontaktierung des Herzmuskels und eine ringförmige zweite Elektrode 2 als Gegenelektrode auf. Der Abstand der beiden Elektroden 1, 2 beträgt ebenfalls 5 mm. Im Gegensatz zu der in Figur 1a dargestellten Elektrodenanordnung weist die halbkugelschalenförmige Elektrode 1 hier eine parallel zu ihrer Symmetrieachse verlaufende kreisförmige Bohrung auf, in die eine stabförmige Elektrode 3 eingesteckt ist.

Zwischen der halbkugelschalenförmigen Elektrode 1 und der ringförmigen Elektrode 2 ist eine weitere Elektrode 4 angeordnet. Das elektrische Potential dieser Elektrode 4 ergibt sich bei der Messung aus dem zwischen den Elektroden 3, 4 fließenden Strom, ist also nicht festgelegt. Diese Elektrode 4 wird deshalb auch als indifferente Elektrode bezeichnet.

Im Gegensatz dazu wird das Potential der halbkugelschalenförmigen Elektrode 1 und der an dieser befestigten stabförmigen Elektrode 3 durch das Potential des Herzmuskels an der Kontaktstelle bestimmt und ist deshalb festgelegt. Diese Elektroden 1, 3 werden deshalb als differente Elektroden bezeichnet.

Die halbkugelschalenförmige Elektrode 1 und die ringförmige Elektrode 2 weisen - wie bei der in Figur 1a dargestellten Elektrodenanordnung - eine fraktale Oberflächenstruktur auf. Hierdurch wird vorteilhaft die aktive Oberfläche der Elektrode 1, 2 und damit die Phasengrenzkapazität der Elektrode 1, 2 vergrößert und so die Phasengrenzimpedanz verringert.

Figur 1c zeigt als drittes Ausführungsbeispiel der Elektrodenanordnung eine 7-polige Elektrodenanordnung mit einer halbkugelschalenförmigen Elektrode 1 zur Kontaktierung des Herzmuskels und vier ringförmigen Gegenelektroden 2a bis 2d, die jeweils eine fraktale Oberflächenstruktur aufweisen, wodurch die aktive Oberfläche der Elektroden 1 und 2a bis 2d und damit deren Phasengrenzkapazität vergrößert wird. Die halbkugelschalenförmige Elektrode 1 und die vier ringförmigen Gegenelektroden 2a bis 2d sind so angeordnet, dass ihre Symmetrieachsen im wesentlichen fluchten.

Die halbkugelschalenförmige Elektrode 1 weist - wie in Figur 1c dargestellt - mittig eine parallel zur Symmetrieachse verlaufende Bohrung zur Aufnahme einer stabförmigen Elektrode 3 auf. Zwischen der halbkugelschalenförmigen Elektrode 1 und der zu dieser unmittelbar benachbarten ringförmigen Elektrode 2a ist mittig eine weitere Elektrode 4 angeordnet.

Figur 1d zeigt schließlich eine vierpolige Elektrodenanordnung mit zwei ringförmigen Elektroden 2a', 2b', einer halbkugelschalförmigen Elektrode 1 sowie einer stabförmigen Elektrode 3.

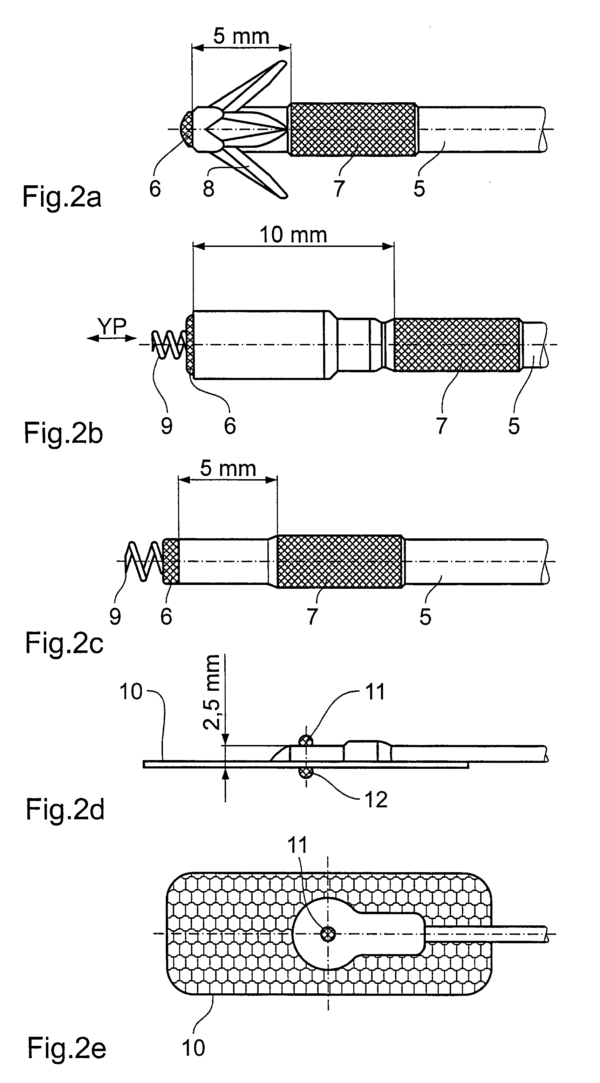

Weitere Elektrodenanordnungen sind in den Figuren 2a bis 2e dargestellt.

Figur 2a zeigt eine bipolare endokardiale Elektrodenanordnung zur Erfassung des monophasischen Aktionspotentials (MAP), die einen schaftartigen Träger 5 zylindrischen Querschnitts aufweist, der die beiden Elektroden 6, 7 aufnimmt und zur Isolierung der beiden Elektroden 6, 7 aus elektrisch isolierendem Material besteht.

An der Stirnseite des Trägers 5 befindet sich die zur Kontaktierung des Herzmuskels vorgesehene differente Elektrode 6, die halbkugelförmig ausgebildet ist und an ihrer den Herzmuskel kontaktierenden Außenfläche mit einer fraktalen Beschichtung versehen ist, um den Übergangswiderstand zwischen Elektrode 6 und Herzmuskel herabzusetzen.

Die indifferente Elektrode 7 dient dagegen zur Kontaktierung des den Herzmuskel umgebenden Gewebes und ist in einem Abstand von ca. 5 mm zu der differenten Elektrode 6 an der Mantelfläche des Trägers 5, diesen ringförmig umhüllend, angeordnet. Die Außenfläche der indifferenten Elektrode 7 ist ebenfalls mit einer fraktalen Beschichtung versehen, um den Übergangswiderstand herabzusetzen und somit eine gute Kontaktierung des Herzmuskels zu ermöglichen.

Die Anordnung der indifferenten Elektrode 7 in einem Abstand von ca. 5 mm zur differenten Elektrode 6 hat sich als vorteilhaft erwiesen, da das MAP bei einer derartigen Anordnung sowohl bei Spontanerregung des Herzens als auch bei einer Stimulation des Herzens die gleiche Morphologie aufweist, was bei einer diagnostischen Auswertung des MAPs von Vorteil ist.

Um die Elektrodenanordnung im implantierten Zustand zu fixieren und eine Wanderung im Körper des Herzschrittmacherträgers zu verhindern sind an dem Träger 5 im Kopfbereich zwischen den beiden Elektroden 6, 7 mehrere Widerhaken 8 angebracht, die in einem Winkel von ca. 30° zur Längsachse des Trägers 5 abgewinkelt sind. Da die Elektrodenanordnung zur Kontaktierung mit dem Kopfbereich in den Herzmuskel eingeführt wird, verhaken sich die Widerhaken 8 im Herzmuskel und verhindern so eine Wanderung der Elektrodenanordnung nach der Implantation. Zum einen wird hierdurch eine gesundheitliche Gefährdung des Herzschrittmacherträgers durch eine Verlagerung der Elektrodenanordnung verhindert. Zum anderen wird hierdurch erreicht, dass der Übergangswiderstand zwischen Herzmuskel und Elektrodenanordnung nach der Implantation weitgehend konstant bleibt, was insbesondere zur Erfassung und Auswertung des MAPs wichtig ist, da die mit einer Verlagerung der Elektrodenanordnung verbundene Änderung des dem Herzschrittmacher zugeführten Messsignals sonst zu Fehldiagnosen führen könnte.

Bei der in Figur 2b dargestellten Elektrodenanordnung erfolgt die Fixierung der Elektrodenanordnung dagegen nicht durch einen Widerhaken wie bei der in Figur 2a dargestellten und vorstehend beschriebenen Elektrodenanordnung, sondern durch einen an der Stirnseite des schaftartigen Trägers 5 angeordneten schraubenfederförmigen Schraubansatz 9, der sich zu seinem freien Ende hin verjüngt und in die Herzwand "eingeschraubt" werden kann. Der Schraubansatz 9 ist hierbei nicht starr mit der Elektrodenanordnung verbunden, sondern ausstoßbar und mittels Mandrin drehbar. Dies ist von entscheidender Bedeutung, da vor dem Ausstoßen des Schraubansatzes 9 MAP-Messungen stattfinden können, die als Kriterium für eine Optimierung der Elektrodenposition dienen können.

Da bei dieser Bauart nur der Schraubansatz 9 in den Herzmuskel eindringt, nicht jedoch - wie bei der in Figur 2a dargestellten Elektrodenanordnung - mindestens ein Teil des Trägers 5, wird der Herzmuskel durch die Implantation der Elektrodenanordnung nur minimal traumatisiert.

Die differente Elektrode 6 ist hierbei ebenfalls an der Stirnseite des Trägers 5 angeordnet und zur Verringerung des Übergangswiderstands mit einer fraktalen Beschichtung versehen, weist jedoch eine weitgehend ebene und damit relativ große Kontaktfläche auf, was vorteilhaft zu einem relativ geringen Übergangswiderstand führt.

Die indifferente Elektrode 7 ist ebenfalls - wie bei der in Figur 2a dargestellten Elektrodenanordnung - ringförmig ausgebildet und zur Verringerung des Übergangswiderstands mit einer fraktalen Beschichtung versehen und umhüllt den zylindrischen Träger 5 schalenförmig in einem Abstand von ca. 10 mm zu der an der Stirnseite angebrachten differenten Elektrode.

Eine weitere Ausführungsform einer derartigen bipolaren Elektrodenanordnung ist in Figur 2c dargestellt.

Die Fixierung der Elektrodenanordnung erfolgt hierbei - wie bei der in Figur 2b gezeigten Elektrodenanordnung - durch einen an der Stirnseite der Elektrodenanordnung angeordneten Schraubansatz 9, der jedoch - im Gegensatz zu der in Figur 2b gezeigten Elektrodenanordnung fest mit der Elektrodenanordnung verbunden ist.

Ein weiterer Unterschied zu der in Figur 2b dargestellten Elektrodenanordnung besteht darin, dass die differente Elektrode 6 in einem Abstand von nur 5 mm zur indifferenten Elektrode 7 angeordnet ist, was den Vorteil bietet, dass das MAP bei Spontanerregung des Herzens dieselbe Morphologie zeigt wie bei einer künstlichen Stimulation durch den Herzschrittmacher, was bei einer diagnostischer Auswertung des MAPs beispielsweise zur Aktivitätssteuerung des Herzschrittmachers vorteilhaft ist.

Ein weiterer Unterschied ist schließlich in der Form der an der Stirnseite des Trägers 5 angeordneten differenten Elektrode 6 zu sehen, die im Gegensatz zu der in Figur 2b dargestellten Elektrodenanordnung topfförmig ist und eine wesentlich größere axiale Erstrekkung aufweist als eine plattenförmige Elektrode, was in einigen Fällen zu einer Verringerung des Übergangswiderstands führt.

Im Gegensatz zu den in Figur 2a - c dargestellten Elektrodenanordnungen zeigen die Figuren 2d und 2e eine epikardiale Elektrodenanordnung zur temporären Kontaktierung des Myokards, wobei die Elektrodenanordnung zur Anlage an der Myokardaußenwand eine aus elektrisch isolierendem Material bestehende elastische Platte 10 aufweist, aus der die halbkugelförmig ausgebildete differente Elektrode 12 nach innen in den Myokard hineinragt. Die indifferente Elektrode 11 ist dagegen an der Außenseite der Platte 10 angebracht und ebenfalls halbkugelförmig ausgebildet.

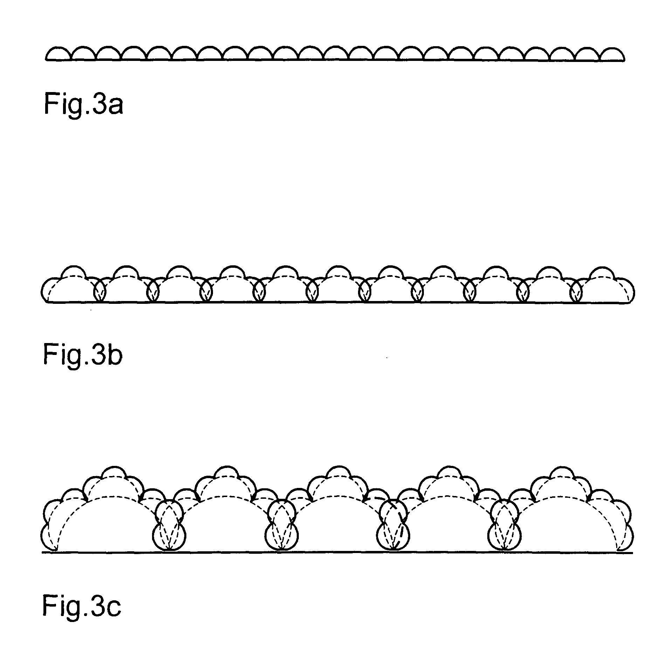

Die Figuren 3a bis 3c geben eine schematische Darstellung zur Verdeutlichung des schrittweisen Aufbaus der Oberflächenstruktur einer fraktalen Elektrode, wie sie bei der Elektrodenanordnung - alternativ zu elektroaktiven Elektroden - vorgesehen ist. Zunächst erfolgt - wie in Figur 3a zu erkennen - eine Ablagerung des auf eine glatte Unterlage (beispielsweise Titan bzw. eine Titanlegierung) abgeschiedenen Beschichtungsmaterials (etwa Iridium) in annähernd halbkugelförmigen, d. h. im Querschnitt halbkreisförmigen Aggregaten in dichter Packung. Die Grundform wird dann - wie die Figuren 3b und 3c erkennen lassen - überlagert durch eine maßstäblich verkleinerte Wiederholung dieser Form in mehreren Stufen. Die verkleinerten Formelemente lagern sich dabei jeweils an der Oberfläche der nächst größeren Unterlage-Form an.

In der Praxis wird eine derartige fraktale Oberflächengeometrie beispielsweise durch geeignete Verfahrensführung eines elektrostatischen Sprühbeschichtungsverfahrens (Sputtern) realisiert. (Im Rahmen dieses kontinuierlichen Verfahrens sind die in den Figuren gezeigten Stufen natürlich räumlich und zeitlich nicht strikt zu trennen.) Als Beschichtungsmaterial sind inerte - d. h. in Körperflüssigkeiten sehr geringe Oxidationsneigung aufweisende - Elemente, Legierungen oder Verbindungen geeignet, die eine fraktale Geometrie ausbilden können, beispielsweise grundsätzlich Elemente, Legierungen oder Nitride, Carbide oder Carbonitride aus der Gruppe Iridium, Platin, Gold oder auch Kohlenstoff.

Die starke und hochgradig differenzierte Tiefenstaffelung der nach hinreichender Prozessdauer erhaltenen Struktur führt zu einer wesentlichen Vergrößerung der aktiven Elektrodenoberfläche um einen Faktor von bis zu 1000 und mehr und damit zu einer entsprechenden Verringerung der Phasengrenzimpedanz.

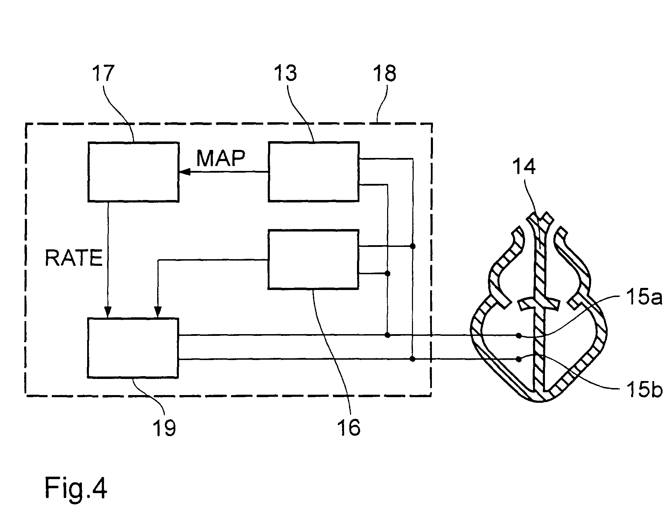

Figur 4 zeigt einen erfindungsgemäßen aktivitätsgesteuerten Herzschrittmacher 18 zur Verwendung mit der in Figur 1a dargestellten Elektrodenanordnung als Blockschaltbild.

Die Stimulierung des Herzens 14 erfolgt über die in Figur 1a detailliert dargestellte bipolare Elektrodenanordnung 15a, 15b, die endokardial angeordnet ist. Die Steuerung erfolgt dabei nach dem sogenannten Demand-Prinzip, d. h. der Herzschrittmacher 18 generiert nur dann einen Stimulationsimpuls und gibt diesen auf das Herz 14, wenn eine natürliche Stimulation des Herzens 14 innerhalb einer bestimmten Zeit ausbleibt. Der Herzschrittmacher 18 weist deshalb eine Eingangsstufe 16 auf, die über die Elektrodenanordnung 15a, 15b laufend ein Elektrokardiogramm (EKG) am Herzen 14 abnimmt und daraus ermittelt, ob eine natürliche Stimulation des Herzens 14 stattfindet oder nicht. Falls die Eingangsstufe 16 eine spontane Herzaktion detektiert, so gibt die Eingangsstufe 16 ein Inhibierungssignal an den Impulsgenerator 19, dessen interne Zeitgeber daraufhin zurückgesetzt werden, sodass die Abgabe eines Stimulationsimpulses unterbleibt.

Der Herzschrittmacher 18 gibt - wie oben beschrieben - nur dann einen Stimulationsimpuls auf das Herz 14, wenn innerhalb einer bestimmten Zeit keine natürliche Stimulation einsetzt. Die Zeit, die der Herzschrittmacher 18 bis zur künstlichen Stimulation des Herzens 14 wartet, ist dabei abhängig von der eingestellten adaptiven Herzrate. Bei einer Belastung des Herzschrittmacherträgers sollte der Herzschrittmacher 18 jedoch eine höhere Herzrate einstellen. Es ist deshalb eine weitere Eingangsstufe 13 vorgesehen, die laufend das an der Elektrodenanordnung 15a, 15b anliegende elektrische Signal misst und daraus das MAP isoliert.

Dieser Eingangsstufe 13 nachgeschaltet ist eine Recheneinheit 17, die in Abhängigkeit von dem MAP die physiologisch angepasste Herzrate RATE bestimmt und den Impulsgenerator 19 entsprechend ansteuert. Die Ansteuerung des Impulsgenerators 19 erfolgt hierbei in der Weise, dass das MAP weitgehend konstant bleibt.

Durch die Aktivitätssteuerung des Herzschrittmachers 18 wird der Herzschrittmacherträger unter Berücksichtigung der jeweiligen Belastung stets mit der physiologisch sinnvollen Herzrate stimuliert.

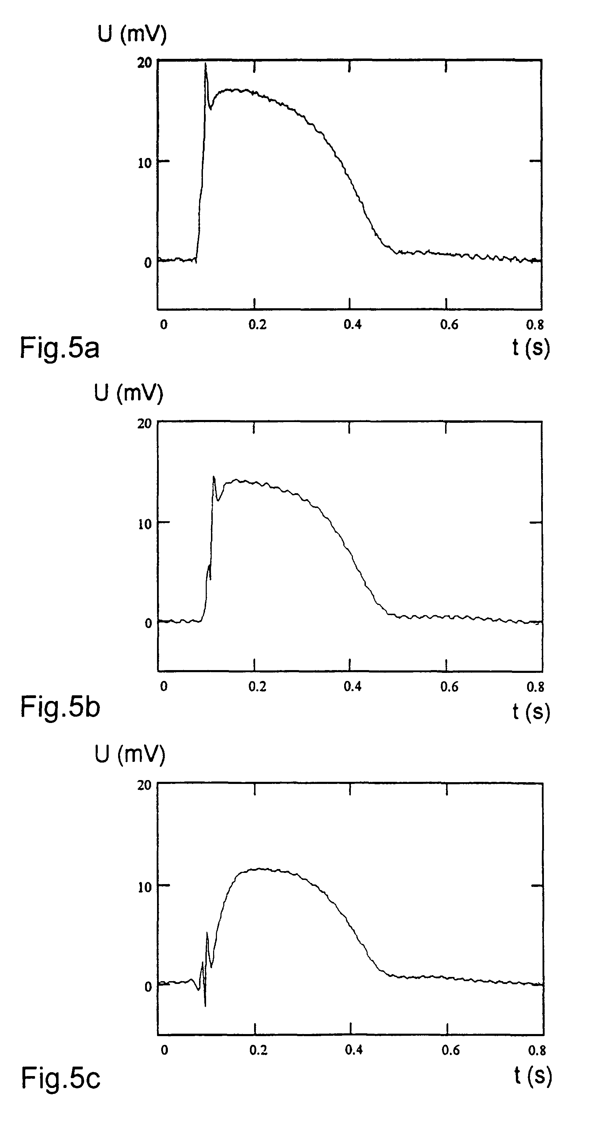

In Figur 5a bis 5f sind Beispiele für die Zeitabhängigkeit der gemessenen Spannung bei der Erfassung des atrialen monophasischen Aktionspotentials MAP gezeigt, die mittels fraktaler Elektroden in verschiedenen Anordnungen aufgenommen wurden.

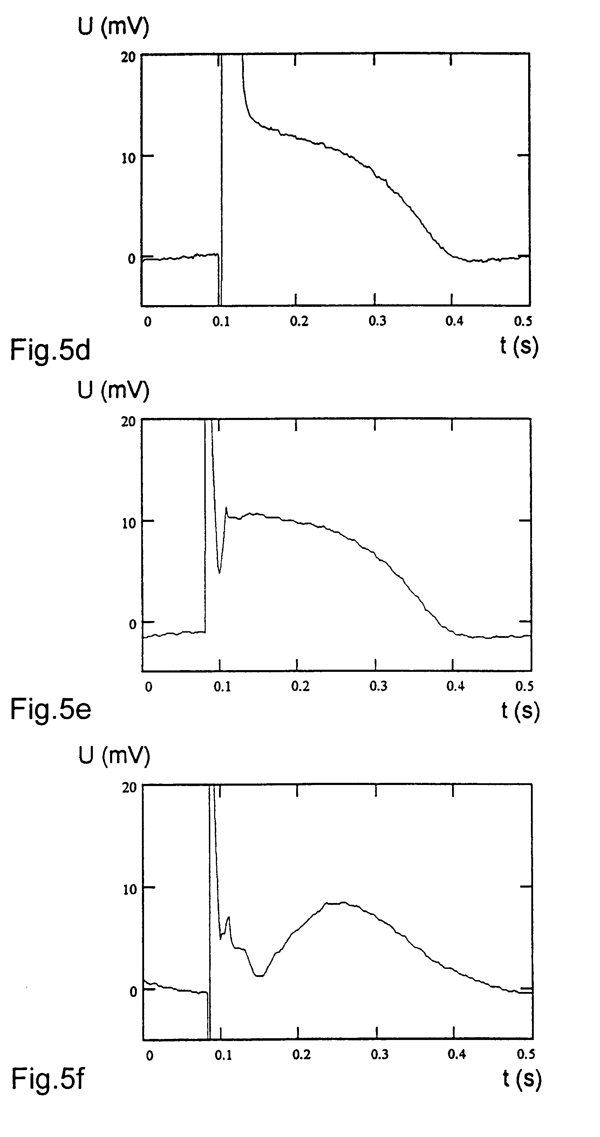

Figur 5a bis 5c zeigen Verläufe spontan erregter MAP, die bei Elektrodenabständen einer differenten zu einer indifferenten fraktalen Elektrode von 4 mm (Figur 5a) bzw. 16 mm (Figur 5b) bzw. unter Anordnung einer indifferenten Elektrode in der Vena subclavia (Figur 5c) aufgenommen wurden, während Figur 5d bis 5f die Potentialverläufe der durch Stimulation mit 140 min$^{-1}$, U = 2 V über die Elektrodenkombination differente fraktale Elektrode/ indifferente fraktale Elektrode in 4 mm Abstand evozierten MAP mit den Figuren 5a bis 5c entsprechenden Erfassungs-Elektrodenanordnungen zeigen. Die charakteristische Morphologie des MAP ist besonders gut in Figur 5a, 5b bzw. 5d, 5e zu erkennen; bei größeren Elektrodenabständen bzw. - wie in Figur 5c und 5f - bei Anordnung der indifferenten Elektrode in der Vena subclavia tritt tendenziell eine Änderung der Morphologie hin zu einem intrakardialen EKG (IEKG) bzw. einem herkömmlichen evozierten Potential auf.

Physikalisch stellt das MAP ein Summenpotential dar, das sich aus der Überlagerung von Transmembranpotentialen in Elektrodennähe ergibt und somit physiologisch die Aktivität des autonomen Nervensystems (ANS) reflektiert, wobei in das MAP-Signal aufgrund der Messung im Vorhof sowohl Sympathikus- als auch Parasympathikusaktivität Eingang findet. Es stellt damit eine wertvolle Informationsquelle sowohl für eine frequenzadaptive Herzstimulation als auch zur Früherkennung von atrialen (speziell tachykarden) Arrhytmien dar und ist somit nach Erkenntnis der Erfinder als Regelgröße eines ratenadaptiven Schrittmachers ebenso geeignet wie als Messgröße für die Kartierung (das Mapping) und ggf. Zerstörung von potentiell arrhythmieerzeugenden Reizleitungsstrukturen im Herzgewebe.

Die Erfindung lässt sich insbesondere bei verschiedenen Arten der Stimulation menschlichen und tierischen Gewebes verwenden. Die Anwendung bei der Herzstimulation ist nicht auf die Stimulation einer Kammer beschränkt. Bevorzugt ist auch die Verwendung in Zweikammer (DDD-)Schrittmachern möglich, bei denen eine MAP-Signal-Aufnahme in einer oder beiden Kammern erfolgt. Die Messung kann auch im Atrium erfolgen.

**Patentansprüche**

1. Stimulationssystem mit einer Elektrodenanordnung sowie einem Impulsgenerator (19), der ausgangsseitig zur Abgabe von Stimulationsimpulsen an das Gewebe (14) mit der Elektrodenanordnung (15a, 15b) verbunden ist und zur Aufnahme eines das Stimulationsverhalten beeinflussenden Steuersignals (RATE) einen Steuereingang aufweist, und einer Eingangsstufe (13, 17), die eingangsseitig zur Aufnahme eines eine Zustandsgröße des Organismus widerspiegelnden elektrischen Messsignals mit der Elektrodenanordnung (15a, 15b) und ausgangsseitig zur Erzeugung des das Stimulationsverhalten des Impulsgenerators (19) beeinflussenden Steuersignals (RATE) in Abhängigkeit von dem Messsignal mit dem Steuereingang des Impulsgenerators (19) verbunden ist, **dadurch gekennzeichnet, dass** Mittel (13) vorgesehen sind zur Ermittlung des monophasischen Aktionspotentials (MAP) aus dem über die Elektrodenanordnung (15a, 15b) abgenommenen Messsignal und zur Erzeugung des das Stimulationsverhalten des Impulsgenerators (19) beeinflussenden Steuersignals (RATE) in Abhängigkeit von dem monophasischen Aktionspotential (MAP).

2. Stimulationssystem nach Anspruch 1, **dadurch gekennzeichnet, dass** es sich bei dem Stimulationssystem um einen Zweikammer-Schrittmacher handelt, der sowohl atriale als ventrikuläre MAP-Signale auswertet.

3. Stimulationssystem nach Anspruch 2, **dadurch gekennzeichnet, dass** die Mittel (13) zur Ermittlung des monophasischen Aktionspotentials (MAP) aus dem über die beiden Elektroden aufgenommenen Messsignal ein Bandpassfilter oder ein Tiefpassfilter aufweisen.

4. Stimulationssystem nach Anspruch 3, **dadurch gekennzeichnet, dass** die untere Grenzfrequenz des Bandpassfilters oder des Tiefpassfilters zwischen 0,1 und 0,5 Hz, insbesondere zwischen 0,25 und 0,5 Hz, gelegen ist.

5. Stimulationssystem nach einem der vorhergehenden Ansprüche 1 bis 3, **dadurch gekennzeichnet, dass** Mittel vorgesehen sind zur Bestimmung der Zeitdauer des im Atrium abgenommenen monophasischen Aktionspotentials, dass eine Zuordnungseinheit vorgesehen ist, die jedem Wert der Zeitdauer des im Atrium abgenommenen monophasischen Aktionspotentials eine Stimulationsrate oder einen Änderungswert der Stimulationsrate zuordnet und ein entsprechendes Steuersignal zur Einstellung der Stimulationsrate des Impulsgenerators (19) erzeugt.

6. Stimulationssystem nach einem der vorhergehenden Ansprüche, **dadurch gekennzeichnet, dass** die Eingangsstufe zur Erfassung der Morphologie des monophasischen Aktionspotentials (MAP) eine Abtastschaltung zur Erzeugung von Abtastwerten des monophasischen Aktionspotentials (MAP) aufweist, dass die Eingangsstufe eine mit der Abtastschaltung verbundene Vergleichereinheit aufweist zum Vergleich der Abtastwerte untereinander und/ oder mit vorgegebenen Vergleichswerten und zur Erzeugung des das Stimulationsverhalten beeinflussenden Steuersignals (RATE).

7. Stimulationssystem nach einem der Ansprüche 1 bis 6, **dadurch gekennzeichnet, dass** die Elektrodenanordnung eine erste Elektrode (1, 15a) zur unmittelbaren Kontaktierung des Gewebes und eine zur ersten Elektrode (1, 15a) elektrisch isolierte zweite Elektrode (2, 15b) zur elektrischen Kontaktierung des Gewebes in der Umgebung der ersten Elektrode (1, 15a) umfasst, wobei mindestens eine der beiden Elektroden (1, 2, 15a, 15b) zur Verringerung der Phasengrenzimpedanz und des nach der Abgabe eines Impulses an das Gewebe auftretenden Nachpotentials zur Ermöglichung der Messung des monophasischen Aktionspotentials eine fraktale Oberflächenstruktur und/ oder eine elektroaktive Beschichtung aufweist.

8. Stimulationssystem nach Anspruch 7, **dadurch gekennzeichnet, dass** die elektroaktive Beschichtung iridiumoxidhaltig ist.

9. Stimulationssystem nach Anspruch 7 oder 8, **dadurch gekennzeichnet, dass** der Abstand zwischen der ersten Elektrode (1, 15a) und der zweiten Elektrode (2, 15b) im wesentlichen fünf Millimeter beträgt.

10. Stimulationssystem nach einem der Ansprüche 7 bis 9, **dadurch gekennzeichnet, dass** die erste Elektrode (1, 15a) und die zweite Elektrode symmetrisch zu jeweils einer Symmetrieachse ausgebildet sind.

**11.** Stimulationssystem nach Anspruch 10, **dadurch gekennzeichnet, dass** die erste Elektrode (1, 15a) und die zweite Elektrode (2, 15b) so angeordnet sind, dass deren Symmetrieachsen im wesentlichen fluchten.

**12.** Stimulationssystem nach Anspruch 10 oder 11, **dadurch gekennzeichnet, dass** die erste Elektrode (1, 15a) und/ oder die zweite Elektrode (2, 15b) zylindrisch oder hohlzylindrisch ausgebildet ist.

**13.** Stimulationssystem nach einem der Ansprüche 7 bis 12, **dadurch gekennzeichnet, dass** die Länge der ersten Elektrode (1, 15a) und/ oder der zweiten Elektrode (2, 15b) im wesentlichen sechs Millimeter beträgt.

**14.** Stimulationssystem nach einem der Ansprüche 7 bis 13, **dadurch gekennzeichnet, dass** die erste Elektrode (1, 15a) und/ oder die zweite Elektrode (2, 15b) im wesentlichen die Form eines Kugelschnitts oder eines Kugelschalenschnitts aufweist.

**15.** Stimulationssystem nach einem der Ansprüche 7 bis 14, **dadurch gekennzeichnet, dass** die erste Elektrode (1, 15a) und/ oder die zweite Elektrode (2, 15b) im wesentlichen aus einem Träger und einer eine fraktale Oberflächenstruktur aufweisenden Beschichtung bestehen.

**16.** Stimulationssystem nach Anspruch 15, **dadurch gekennzeichnet, dass** der Träger im wesentlichen aus Titan besteht.

**17.** Stimulationssystem nach Anspruch 15 oder 16, **dadurch gekennzeichnet, dass** die eine fraktale Oberflächenstruktur aufweisende Beschichtung iridiumhaltig ist.

**18.** Stimulationssystem nach einem der vorhergehenden Ansprüche, **dadurch gekennzeichnet, dass** zur Analyse des monophasischen Aktionspotentials zwecks Erkennung einer sich anbahnenden oder bereits bestehenden Tachykardie eine Signalverarbeitungseinheit vorgesehen ist, die zur Abgabe eines den Tachykardiezustand anzeigenden Steuersignals einen Steuerausgang aufweist, dass der Impulsgenerator (19) eine Betriebsart zur Tachykardieprävention oder -bekämpfung aufweist, die über einen Steuereingang aktivierbar ist, der mit dem Steuerausgang der Signalverarbeitungseinheit verbunden ist.

**Claims**

**1.** A stimulation system having an electrode arrangement and also a pulse generator (19), which at the output side is connected to the electrode arrangement (15a, 15b) for the emission of stimulation pulses to the tissue (14) and comprises a control input for recording a control signal (RATE) which influences the stimulation behaviour, and having an input stage (13, 17), which on the input side is connected to the electrode arrangement (15a, 15b) for recording an electrical measuring signal reflecting a state variable of the organism and on the output side is connected to the control input of the pulse generator (19) for generating the control signal (RATE) which influences the stimulation behaviour in dependence on the measuring signal,
**characterised in that** means (13) are provided for the determination of the monophasic action potential (MAP) from the measuring signal obtained via the electrode arrangement (15a, 15b) and for the generation of the control signal (RATE) influencing the stimulation behaviour of te pulse generator (19) from the monophasic action potential (MAP).

**2.** A stimulation system according to Claim 1, **characterised in that** the stimulation system is a two-chamber pacemaker which evaluates both atrial and ventricular MAP signals.

**3.** A stimulation system according to Claim 2, **characterised in that** the means (13) for determining the monophasic action potential (MAP) from the measuring signal recorded via the two electrodes comprise a bandpass filter or a lowpass filter.

**4.** A stimulation system according to Claim 3, **characterised in that** the lower cutoff frequency of the bandpass filter or of the lowpass filter is between 0.1 and 0.5 Hz, in particular between 0.25 and 0.5 Hz.

**5.** A stimulation system according to one of the preceding Claims,
**characterised in that** means are provided for determining the duration of the monophasic action potential obtained in the atrium,
**in that** a assignment unit is provided, which assigns a stimulation rate or a modification value of the stimulation rate to each value of the duration of the monophasic action potential obtained in the atrium and generates a corresponding control signal for adjusting the stimulation rate of the pulse generator (19).

**6.** A stimulation system according to one of the preceding Claims,
**characterised in that** the input stage for detecting the morphology of the monophasic action potential (MAP) comprises a sampling circuit for generating sampling values of the monophasic action potential (MAP),

**in that** the input stage comprises a comparator unit connected to the sampling circuit for the comparison of the sampling values with one another and/or with predetermined comparison values and for generating the control signal influencing the stimulation behaviour (RATE).

7. A stimulation system according to one of Claims 1 to 6,
**characterised in that** the electrode arrangement comprises a first electrode (1, 15a) for the direct contacting of the tissue and a second electrode (2, 15b) electrically insulated from the first electrode (1, 15a) for the electrical contacting of the tissue in the vicinity of the first electrode (1, 15a), wherein at least one of the two electrodes (1, 2, 15a, 15b) comprises a fractal surface structure and/or an electroactive coating for the reduction of the phase limit impedance and the after-potential occurring after the emission of a pulse to the tissue to enable the measurement of the monophasic action potential.

8. A stimulation system according to Claim 7,
**characterised in that** the electroactive coating contains iridium.

9. A stimulation system according to Claim 7 or 8,
**characterised in that** the distance between the first electrode (1, 15a) and the second electrode (2, 15b) is substantially five millimetres.

10. A stimulation system according to one of Claims 7 to 9,
**characterised in that** the first electrode (1, 15a) and the second electrode (2, 15b) are constructed in each case symmetrically to an axis of symmetry.

11. A stimulation system according to Claim 10,
**characterised in that** the first electrode (1, 15a) and the second electrode (2, 15b) are arranged so that their axes of symmetry substantially align.

12. A stimulation system according to Claim 10 or 11,
**characterised in that** the first electrode (1, 15a) and/or the second electrode (2, 15b) has a cylindrical or hollow cylindrical construction.

13. A stimulation system according to one of Claims 7 to 12,
**characterised in that** the length of the first electrode (1, 15a) and/or of the second electrode (2, 15b) is substantially six millimetres.

14. A stimulation system according to one of Claims 7 to 13,
**characterised in that** the first electrode (1, 15a) and/or the second electrode (2, 15b) substantially takes the shape of a spherical section or a spherical shell section.

15. A stimulation system according to one of Claims 7 to 14,
**characterised in that** the first electrode (1, 15a) and/or the second electrode (2, 15b) essentially consist of a substrate and a coating having a fractal surface.

16. A stimulation system according to Claim 15,
**characterised in that** the substrate is substantially made from titanium.

17. A stimulation system according to Claim 15 or 16,
**characterised in that** the coating comprising a fractal surface structure contains iridium.

18. A stimulation system according to one of the preceding Claims,
**characterised in that** for the analysis of the monophasic action potential for the purpose of diagnosing a developing or already existing tachycardia there is provided a signal processing unit, which for the emission of a control signal indicating the tachycardiac condition comprises a control output, **in that** the pulse generator (19) comprises an operating mode for the prevention or control of tachycardia, which can be activated via a control input which is connected to the control output of the signal processing unit.

**Revendications**

1. Système de stimulation comportant un dispositif à électrodes ainsi qu'un générateur d'impulsions (19), qui est relié côté sortie de manière à délivrer des impulsions de stimulation au tissu (14) avec le dispositif à électrodes (15a,15b) et comporte une entrée de commande pour la réception d'un signal de commande (RATE) influençant le comportement de stimulation, et un étage d'entrée (13,17), qui est relié côté entrée pour la réception d'un signal de mesure électrique reproduisant une grandeur d'état de l'organisme, au dispositif à électrodes (15a,15b) et est relié côté sortie, pour la production du signal de commande (RATE) influençant le procédé de stimulation du générateur d'impulsions (19), en fonction du signal de mesure, à l'entrée de commande du générateur d'impulsions (19), **caractérisé en ce que** des moyens (13) sont prévus pour déterminer le potentiel d'action monophasé (MAP) à partir du signal de mesure prélevé par l'intermédiaire du dispositif à électrodes (15a,15b), et pour produire le signal de commande (RATE), qui influe sur le procédé de stimulation du générateur d'impulsions (19), en fonction du potentiel d'action monophasé (MAP).

**2.** Système de stimulation selon la revendication 1, **caractérisé en ce qu'**en ce qui concerne le système de stimulation, il s'agit d'un stimulateur à deux chambres, qui évalue aussi bien des signaux MAP atriaux que des signaux MAP ventriculaires.

**3.** Système de stimulation selon la revendication 2, **caractérisé en ce que** les moyens (13) pour déterminer le potentiel d'action monophasé (MAP) à partir du signal de mesure enregistré par l'intermédiaire des deux électrodes possèdent un filtre passe-bande ou un filtre passe-bas.

**4.** Système de stimulation selon la revendication 3, **caractérisé en ce que** la fréquence limite inférieure du filtre passe-bande ou du filtre passe-bas est située entre 0,1 et 0,5 Hz, notamment entre 0,25 et 0,5 Hz.

**5.** Système de stimulation selon l'une des revendications précédentes 1 à 3, **caractérisé en ce que** des moyens sont prévus pour déterminer la durée du potentiel d'action monophasée prélevée dans l'oreillette, qu'il est prévu une unité d'association, qui associe à chaque valeur de la durée du potentiel d'action monophasé prélevé dans l'oreillette, une cadence de stimulation ou une valeur de variation de la cadence de stimulation et produit un signal de commande correspondant pour le réglage de la cadence de stimulation du générateur d'impulsions (19).

**6.** Système de stimulation selon l'une des revendications précédentes, **caractérisé en ce que** l'étage d'entrée servant à détecter la morphologie du potentiel d'action monophasé (MAP) comporte un circuit d'échantillonnage pour produire des valeurs d'échantillonnage du potentiel d'action monophasé (MAP), que l'étage d'entrée comporte une unité de comparaison reliée au circuit d'échantillonnage et qui sert à comparer les valeurs d'échantillonnage entre elles et/ou à des valeurs de comparaison prédéterminées et à produire le signal de commande (RATE) qui influe sur le comportement de stimulation.

**7.** Système de stimulation selon l'une des revendications 1 à 6, **caractérisé en ce que** le dispositif à électrodes comprend une première électrode (1,15a) destinée à venir en contact direct avec le tissu et une seconde électrode (2,15b), qui est isolée électriquement par rapport à la première électrode (1,15a) et sert à établir un contact électrique avec le tissu dans l'environnement de la première électrode (1,15a), et dans lequel au moins l'une des deux électrodes (1,2,15a,15b) servant à réduire l'impédance limite de phase et le potentiel d'arrière-onde, qui apparaît après la délivrance d'une impulsion au tissu, pour permettre la mesure du potentiel d'action monophasé, comporte une structure superficielle fractale et/ou un revêtement électro-actif.

**8.** Système de stimulation selon la revendication 7, **caractérisé en ce que** le revêtement électro-actif contient de l'oxyde d'iridium.

**9.** Système de stimulation selon la revendication 7 ou 8, **caractérisé en ce que** la distance entre la première électrode (1,15a) et la seconde électrode (2,15b) est égale essentiellement à cinq millimètres.

**10.** Système de stimulation selon l'une des revendications 7 à 9, **caractérisé en ce que** la première électrode (1,15a) et la seconde électrode sont agencées symétriquement par rapport à un axe de symétrie respectif.

**11.** Système de stimulation selon la revendication 10, **caractérisé en ce que** la première électrode (1,15a) et la seconde électrode (2,15b) sont disposées de telle sorte que leurs axes de symétrie sont essentiellement alignés.

**12.** Système de stimulation selon la revendication 10 ou 11, **caractérisé en ce que** la première électrode (1,15a) et/ou la seconde électrode (2,15b) est agencée avec une forme cylindrique ou cylindrique creuse.

**13.** Système de stimulation selon l'une des revendications 7 à 12, **caractérisé en ce que** la longueur de la première électrode (1,15a) et/ou de la seconde électrode (2,15b) est égale essentiellement à six millimètres.

**14.** Système de stimulation selon l'une des revendications 7 à 13, **caractérisé en ce que** la première électrode (1,15a) et/ou la seconde électrode (2,15b) possède essentiellement la forme d'une section de sphère ou d'une section de coque sphérique.

**15.** Système de stimulation selon l'une des revendications 7 à 14, **caractérisé en ce que** la première électrode (1,15a) et/ou la seconde électrode (2,15b) sont constituées essentiellement par un support et par un revêtement possédant une structure superficielle fractale.

**16.** Système de stimulation selon la revendication 15, **caractérisé en ce que** le support est constitué essentiellement de titane.

**17.** Système de stimulation selon la revendication 15 ou 16, **caractérisé en ce que** le revêtement possédant une couche superficielle fractale contient de l'iridium.

18. Système de stimulation selon l'une des revendications précédentes, **caractérisé en ce que** pour l'analyse du potentiel d'action monophasé en vue d'identifier une tachycardie commençante ou déjà existante, il est prévu une unité de traitement de signaux, qui, pour la délivrance d'un signal de commande affichant l'état de tachycardie, possède une sortie de commande, que le générateur d'impulsions (19) possède un type de fonctionnement destiné à empêcher ou combattre la tachycardie et qui peut être activé par l'intermédiaire d'une entrée de commande qui est reliée à la sortie de commande de l'unité de traitement de signaux.

Fig.1a

Fig.1b

Fig.1c

Fig.1d

Fig.2a

Fig.2b

Fig.2c

Fig.2d

Fig.2e

Fig.3a

Fig.3b

Fig.3c

Fig.4

U (mV)

Fig.5a

U (mV)

Fig.5b

U (mV)

Fig.5c

Fig.5d

Fig.5e

Fig.5f